# FASCICULE DE BREVET EUROPEEN

(11) **EP 1 078 085 B1**
(45) Date de publication et mention de la délivrance du brevet: **28.07.2004**
(21) Numéro de dépôt: 99917006.1
(22) Date de dépôt: 07.05.1999
(51) Int. Cl.: C12N 15/82, C07K 14/415, A01N 65/00, C12N 15/29

(54) **UTILISATION D'UN POLYPEPTIDE DERIVE D'UNE ALBUMINE PA1B DE LEGUMINEUSE COMME INSECTICIDE**
VERWENDUNG EINES POLYPEPTIDS AUS HÜLSEN-ALBUMIN (PA1B) ALS INZEKTIZID
USE OF A POLYPEPTIDE DERIVED FROM A PA1B LEGUME ALBUMEN AS INSECTICIDE

(30) Priorité: 11.05.1998 FR 9805877
(43) Date de publication de la demande: 28.02.2001
(73) Titulaire: Institut National de la Recherche Agronomique (I.N.R.A.), 75338 Paris (FR); Institut National des Sciences Appliquées de Lyon, 69621 Villeurbanne (FR)
(72) Inventeur: DELOBEL, Bernard, F-69100 Villeurbanne (FR); GRENIER, Annie, F-69680 Chassieu (FR); GUEGUEN, J., La Croix de la Bauche, F-44240 La Chapelle sur Erdre (FR); FERRASSON, Eric, F-44300 Nantes (FR); MBAILAO, Mbaiguinam, N'Djamena (TD)
(74) Mandataire: Vialle-Presles, Marie José
(86) Numéro de dépôt international: PCT/FR1999/001085
(87) Numéro de publication internationale: WO 1999/058695

(56) Documents cités:
- WO-A-93/04586
- HIGGINS T J V ET AL: "Gene structure, protein structure and regulation of the synthesis of a sulfur-rich protein in pea seeds" JOURNAL OF BIOLOGICAL CHEMISTRY. (MICROFILMS), vol. 261, no. 24, 25 août 1986 (1986-08-25), pages 11124-11130, XP002091347 MD US cité dans la demande
- BAKER J E ET AL: "Multiplicity of albumins from wheat inhibitory to amylase from the rice weevil coleoptera curculionidae" JOURNAL OF ECONOMIC ENTOMOLOGY, vol. 82, no. 6, 1989, pages 1548-1553, XP002091350
- WATANABE Y ET AL: "A peptide that stimulates phosphorylation of the plant insulin-binding protein" THE EUROPEAN JOURNAL OF BIOCHEMISTRY, vol. 224, no. 1, août 1994 (1994-08), pages 167-172, XP002091348 BERLIN DE cité dans la demande
- EALING P M ET AL.: "Expression of the pea albumin gene in transgenic white clover and tobacco" TRANSGENIC RESEARCH, vol. 3, 1994, pages 344-354, XP002091349 US
- BUONOCORE V ET AL.: "Wheat protein inhibitors of alpha amylase" PHYTOCHEMISTRY, vol. 16, no. 7, 1977, pages 811-820, XP002091351 OXFORD GB
- GATEHOUSE A M R ET AL: "IDENTIFYING PROTEINS WITH INSECTICIDAL ACTIVITY: USE OF ENCODING GENES TO PRODUCE INSECT-RESISTANT TRANSGENIC CROPS" PESTICIDE SCIENCE, vol. 52, no. 2, février 1998 (1998-02), pages 165-175, XP000751964

## Description

La présente invention concerne des protéines insecticides et leur utilisation pour la protection des plantes, et en particulier des céréales, de leurs graines et des produits dérivés de celles-ci contre les insectes ravageurs.

Des insectes ravageurs des graines de céréales se rencontrent dans différentes familles, en particulier parmi les Coléoptères, des Lépidoptères et des Homoptères. Chez les Coléoptères, on citera en particulier les charançons des grains (*Sitophilus oryzae, Sitophilus zeamais*, *Sitophilus granarius*), ainsi que *Tenebrio* spp., *Rhyzopertha dominica, Trogoderma* spp., *Tribolium confusum.* Chez les Lépidoptères, on citera en particulier *Sitotroga cerealella* et *Ephestia kuehniella.*

Les ravageurs de graines de céréales sont parmi les principaux ennemis des récoltes qu'ils attaquent au champ (au moins dans les régions chaudes), et surtout dans les silos de conservation ; ils peuvent également attaquer les produits transformés dérivés de céréales (par exemple farines, semoules, etc.) Ces insectes causent des dégâts très importants et sont chaque année à l'origine de la destruction d'une partie importante (pouvant avoisiner 25%) de la récolte mondiale de céréales récoltées chaque année.

Pour lutter contre ces insectes, différentes méthodes ont été préconisées. L'utilisation d'insecticides (LINDANE®, puis MALATHION® et bromure d'éthylène) est actuellement remise en question, du fait des problèmes posés par la présence de résidus de ces produits dans l'alimentation. En outre, des résistances à ces produits sont apparues chez de nombreux insectes cibles, ce qui rend leur utilisation de moins en moins efficace. Pour remplacer ces insecticides ou limiter leur usage, différentes méthodes ont été proposées [pour revue, cf par exemple F.H ARTHUR, J. Stored Prod. Res., 32, pp. 293-302, (1996)]. Les plus développées actuellement sont des méthodes physiques, telles que le refroidissement des silos, la conservation sous CO₂ ou sous azote ; ces méthodes sont toutefois onéreuses, et leur mise en oeuvre, qui nécessite une haute technicité, est délicate ; elles ne sont donc pas applicables partout.

Un autre type d'approche, qui fait l'objet de nombreuses recherches, consiste à produire des plantes transgéniques exprimant un ou plusieurs gène(s) leur conférant une résistance contre l'attaque des insectes. Cependant, cette approche nécessite que l'on dispose de gènes appropriés, qui doivent en outre être acceptables à la fois pour l'environnement et par les consommateurs.

La plupart des insectes présentent une spécificité alimentaire plus ou moins stricte ; c'est ainsi que les graines de céréales sont attaquées par les charançons des grains (*Sitophilus oryzae, Sitophilus zeamais, Sitophilus granarius*), qui n'attaquent pas les graines de légumineuses ; par contre, d'autres ravageurs, tels que les bruches, attaquent les légumineuses, mais pas les céréales.

Des travaux précédents de l'équipe des Inventeurs [DELOBEL et GRENIER, J. Stored Prod. Res., 29, pp. 7-14, (1993)], ont montré que les trois espèces de Sitophilus mentionnées ci-dessus pouvaient se développer sur des châtaignes ou des glands, mais qu'en revanche elles mouraient rapidement sur pois cassés, cette mortalité étant consécutive à la consommation des pois par ces charançons.

Les Inventeurs ont entrepris de rechercher la substance toxique responsable de cette mortalité. Il est par ailleurs connu que les légumineuses renferment plusieurs substances entomotoxiques, et qu'il existe chez diverses espèces d'insectes pour lesquelles les légumineuses sont toxiques, des sous-populations naturelles plus ou moins résistantes à la toxicité des légumineuses.

Par exemple, dans le cas des charançons des grains, un test effectué par l'équipe des Inventeurs sur 90 souches d'origines géographiques différentes, a montré que 4 souches appartenant à l'espèce *Sitophilus oryzae* comportaient des individus capables de survivre sur pois cassés au stade adulte ; en revanche, aucune souche possédant cette faculté n'a été mise en évidence chez les espèces *Sitophilus zeamais,* ou *Sitophilus granarius ;* l'étude du déterminisme génétique de cette résistance a montré que ce caractère est monogénique, récessif et autosomal [GRENIER et al., Heredity, 79, pp. 15-23, (1997)].

Les Inventeurs ont sélectionné une souche de *S. oryzae* homozygote pour ce gène de résistance, et ont utilisé cette souche pour rechercher la substance toxique vis-à-vis de laquelle se manifestait le mécanisme de résistance codé par ce gène.

Les Inventeurs ont ainsi constaté que cette toxicité était associée aux isoformes d'une protéine, de séquence similaire à celle de l'albumine de pois PA1b décrite par HIGGINS et al., [J. Biol. Chem., 261(24), pp. 11124-11130, (1986)], et présentant une forte similitude (65% d'identité) avec la leginsuline de soja [WATANABE et al., Eur. J. Biochem., 15, pp. 224:1-167-72, (1994)]. Aucune propriété entomotoxique n'avait jusqu'à présent été associée à la protéine PA1b, à la leginsuline ou à d'autres protéines homologues.

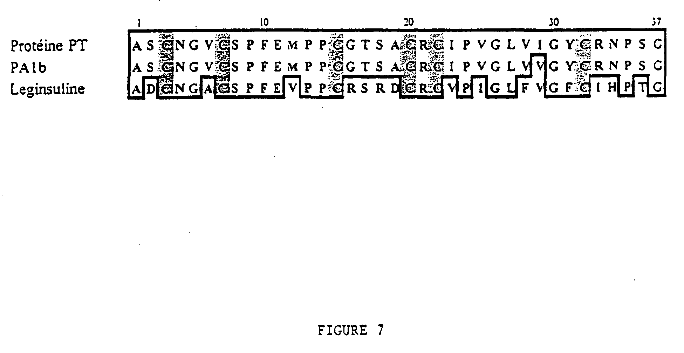

L'alignement de la séquence de l'une des isoformes de la protéine purifiée par les Inventeurs, avec celles de la protéine PA1b de pois, publiée par HIGGINS et al. et de la leginsuline de soja publiée par WATANABE et al., est représentée sur la Figure 7. Ces 3 séquences comportent en particulier 6 résidus cystéines occupant des positions conservées.

La présente invention a pour objet l'utilisation en tant qu'insecticide, d'un polypeptide comprenant une séquence répondant à la formule générale (I) suivante :

X₁CX₂CX₃CX₄CX₅CX₆CX₇ (I)

dans laquelle C représente un résidu cystéine,et
- X₁ répond à la séquence y₁y₂ dans laquelle y₁ et y₂ représentent chacun un acide aminé choisi parmi l'alanine, la sérine, la glycine, et la thréonine, ou bien y₁ représente un acide aminé choisi parmi l'alanine, la sérine, la glycine, et la thréonine, et y₂ représente l'acide glutamique ou l'acide aspartique ; et/ou
- X₂ répond à la séquence y₃y₄y₅ dans laquelle y₃ représente la glutamine ou l'asparagine, et y₄ et y₅ représentent chacun un acide aminé choisi parmi l'alanine, la serine, la glycine, la thréonine, la valine, la leucine, l'isoleucine, et la méthionine ; et/ou
- X₃ répond à la séquence y₆y₇y₈y₉y₁₀y₁₁y₁₂ dans laquelle y₆ représente un acide aminé choisi parmi l'alanine, la sérine, la glycine, et la thréonine, y₇, y₁₁, et y₁₂ représentent chacun la proline, y₈ représente un acide aminé choisi parmi la phénylalanine, le tryptophane, et la tyrosine, y₉ représente l'acide aspartique ou l'acide glutamique, y₁₀ représente un acide aminé choisi parmi la valine, la leucine, l'isoleucine, et la méthionine ; et/ou
- X₄ répond à la séquence y₁₃y₁₄y₁₅y₁₆, dans laquelle y₁₃, y₁₄, y₁₅, y₁₆, représentent chacun un acide aminé choisi parmi l'alanine, la sérine, la glycine, et la thréonine, ou bien y₁₄ représente un acide aminé choisi parmi l'alanine, la sérine, la glycine, et la thréonine, y₁₃ et y₁₅ représentent chacun un acide aminé basique, et y₁₆ représente l'acide aspartique ou l'acide glutamique ; et/ou
- X₅ représente un acide aminé basique ; et/ou
- X₆ répond à la séquence y₁₇y₁₈y₁₉y₂₀y₂₁y₂₂y₂₃y₂₄y₂₅, dans laquelle y₁₇, y₁₉, y₂₁, et y₂₃ représentent chacun un acide aminé choisi parmi la valine, la leucine, l'isoleucine, et la méthionine, y₁₈ représente la proline, y₂₀ et y₂₄ représentent chacun un acide aminé choisi parmi l'alanine, la sérine, la glycine, et la thréonine, y₂₂ représente un acide aminé choisi parmi la valine, la leucine, l'isoleucine, la méthionine, la phénylalanine, le tryptophane, et la tyrosine, et y₂₅ représente un acide aminé choisi parmi la phénylalanine, le tryptophane, et la tyrosine ; et/ou
- X₇ répond à la séquence y₂₆y₂₇y₂₉y₂₉y₃₀ dans laquelle y₂₆ représente un acide aminé basique, ou un acide aminé choisi parmi la valine, la leucine, l'isoleucine, et la méthionine, y₂₇ représente l'asparagine ou la glutamine ou un acide aminé basique, y₂₈ représente la proline, et y₂₉ et y₃₀ représentent chacun un acide aminé choisi parmi l'alanine, la serine. La glycine, et la thréonine.

Selon un mode de mise en oeuvre préféré de la présente invention, le polypeptide utilisé comme insecticide présente au moins 40%, de préférence au moins 60% d'homologie avec une quelconque des isoformes d'une albumine PA1b.

Au sens de la présente invention, on entend par : « albumine PA1b » non seulement toute isoforme de la protéine PA1b de pois, mais également toute protéine de la même famille présente chez d'autres plantes, et pouvant notamment être purifiée à partir de graines de légumineuses, en particulier des légumineuses de la famille des Cesalpinaceae, des Mimosaceae ou des Fabaceae, ou de Méliacées, telles que *Khaya senegalensis.*

Des polypeptides utilisables conformément à l'invention, peuvent être des polypeptides naturels, par exemple les leginsulines de légumineuses, telle que la leginsuline de soja décrite par WATANABE et al. ; il peut s'agir également de polypeptides artificiels dont la séquence est dérivée de celle d'une PA1b par l'addition, la délétion, ou la substitution d'un petit nombre d'acides aminés. On peut par exemple utiliser des polypeptides comprenant une séquence répondant à la formule générale (I), ou une portion de celle-ci correspondant à la région impliquée dans l'activité insecticide. Ce peptide actif peut éventuellement être fusionné à son extrémité N-terminale et/ou à son extrémité C-terminale avec une autre séquence peptidique.

Ces polypeptides peuvent être obtenus par les méthodes classiques, connues en elles-mêmes, par exemple par synthèse peptidique, ou par génie génétique, en exprimant, dans une cellule-hôte appropriée une séquence codant pour le polypeptide souhaité. Ils peuvent également, dans le cas des polypeptides naturels, tels que la PA1b et la leginsuline, être purifiés à partir de graines de plantes telles que des légumineuses ou des Méliacées.

Conformément à l'invention, les polypeptides comprenant une séquence de formule générale (I) peuvent être utilisés en tant que seul principe actif d'un insecticide, ou bien associés à un ou plusieurs autres principes actifs. Ils peuvent être utilisés en particulier, pour lutter contre les insectes ravageurs des graines de céréales, et également contre des insectes phytophages, tels que les lépidoptères *Mamestra brassicae* ou *Ostrinia nubilalis* ou les coléoptères *Chrysomélidae* comme *Phaedon cochleariae* ou *Curculionidae* comme *Anthonomus grandis* ou contre des Insectes phloemophages tels que les pucerons.

En outre, les Inventeurs ont constaté que la protéine PA1b conservait son activité insecticide pendant plusieurs années dans les graines sèches, et que cette activité n'était pas affectée par un chauffage à 100°C.

D'autre part, cette protéine n'est pas toxique pour l'homme ou les animaux supérieurs ; elle est présente dans les légumineuses qui font partie de leur alimentation habituelles.

Les polypeptides de séquence générale (I), sont particulièrement bien adaptés pour la protection, en particulier pendant le stockage, des graines, des farines, ou des produits transformés qui en sont dérivés.

Pour la mise en oeuvre de la présente invention, la concentration du polypeptide de séquence (I) au niveau du produit à protéger (plante, graines, ou produits dérivés) est généralement de 10 µmoles/kg à 100 mmoles/kg (ou de 10µM à 100mM), et avantageusement de 50µmoles/kg à 10 mmoles/kg (ou de 50µM à 10mM).

Selon un mode de mise en oeuvre préféré de la présente invention, on traite le produit à protéger avec une préparation comprenant ledit polypeptide. Celui-ci peut par exemple être sous forme d'une préparation purifiée ou d'une fraction enrichie, qui peuvent notamment être obtenues à partir de graines de plantes produisant naturellement ledit polypeptide, ou bien à partir de cultures de cellules exprimant un gène codant pour ce polypeptide.

Selon un autre mode de mise en oeuvre préféré de la présente invention, on produit une plante transgénique, transformée par au moins un gène codant pour ledit polypeptide, et exprimant ce dernier dans au moins un de ses tissus ou organes.

La présente invention englobe également les plantes transgéniques produites de la sorte ; avantageusement, lesdites plantes sont des céréales.

Ces plantes peuvent être obtenues par les techniques habituelles de transgénèse végétale, qui sont connues en elles-mêmes.

Il est ainsi possible d'obtenir dans une plante une expression ubiquitaire et/ou une expression ou une surexpression dans certains tissus ou organes (par exemple dans les graines) d'un polypeptide de séquence (I), et de ce fait de protéger la plante, le tissu ou l'organe concerné, contre les attaques d'insectes pour lesquels ce polypeptide est toxique. En particulier, l'expression d'un polypeptide de séquence (I) dans les graines permet de protéger celles-ci, même après la récolte, ainsi que les farines et les produits transformés obtenus à partir de ces graines.

La présente invention sera mieux comprise à l'aide du complément de description qui va suivre qui se réfère à des exemples non-limitatifs, décrivant la purification et illustrant les propriétés insecticides d'une albumine PA1b de légumineuse.

### EXEMPLE 1 : MISE EN EVIDENCE DE LA TOXICITÉ DE DIFÉRENTES FARINES DE LEGUMINEUSES POUR DES CHARANCONS DES CÉRÉALES

La toxicité de farines de différentes légumineuses a été testée sur des charançons *(Sitophilus oryzae*). Les expérimentations ont été effectuées en parallèle sur des animaux de type sauvage (souche sensible S), et sur des mutants survivant à l'alimentation sur pois (souche résistante R).

Les charançons (*Sitophilus oryzae*) sont élevés en enceinte régulée à 27,5°C et 70% d'humidité relative. De jeunes adultes âgés d'une semaine sont prélevés dans ces élevages de masse pour les tests. Pour chaque test, des lots de 30 insectes sont mis en expérimentation, et la mortalité journalière est notée.

Des boulettes de farine sont pétries avec de l'eau, mises à sécher 24 h, et utilisées pour l'alimentation des charançons. La farine grise de blé utilisée est complétée avec différentes proportions de farine de légumineuse, tamisée à 0,2 mm d'ouverture de maille.

Les courbes dose-réponse de mortalité des charançons ont été obtenues en utilisant différentes doses de chaque farine à tester. Les résultats sont traités par le programme « Toxicologie » [FEBVAY et RAHBÉ, « Toxicologie », un programme pour l'analyse des courbes de mortalité par la méthode des probits sur MacIntosh, Cahiers Techn. INRA, 27, pp. 77-78 (1991)]. Ce programme utilise la transformation en Probits des mortalités cumulées et détermine l'équation de la courbe de régression et la concentration létale 50%. Ces valeurs sont déterminées après 4 et 7 jours d'exposition.

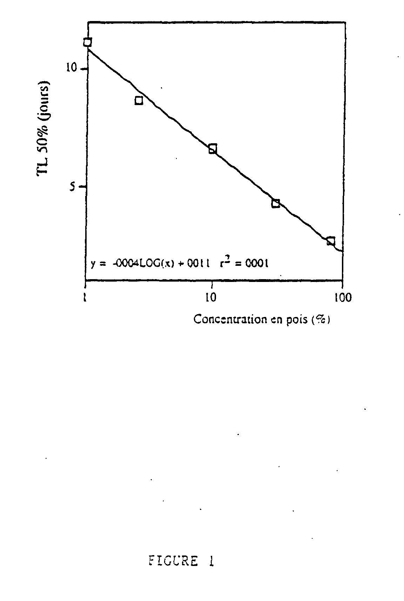

En outre, pour chaque concentration de farine de pois, les temps létaux 50% (TL50) pour la souche sensible S ont également été calculés. La courbe d'étalonnage ainsi établie permettra de déterminer dans la suite des expérimentations, pour chaque farine ou fraction de farine testée, la concentration équivalente en farine de pois (en % de pois dans le blé). Cette courbe est représentée sur la Figure 1.

### Toxicité de la farine de pois :

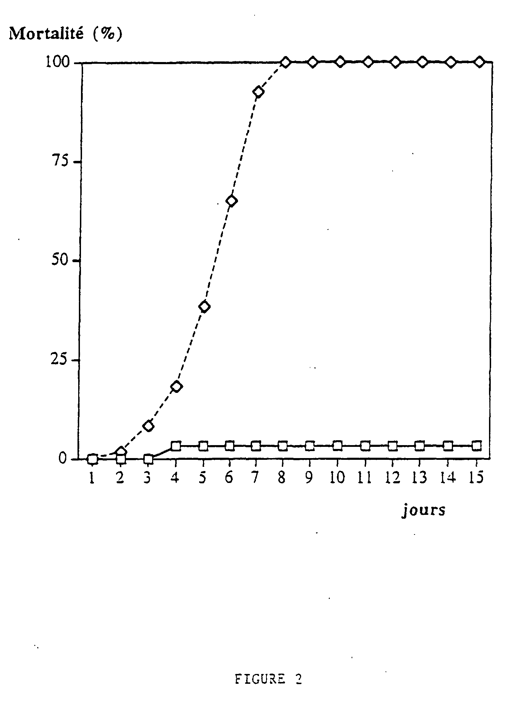

La Figure 2 représente la mortalité cumulée pour des adultes de la souche sensible S de *Sitophilus oryzae*, sur pois (◇), et sur blé (□), en fonction du temps d'alimentation en jours. Ces résultats montrent que les charançons des céréales sont rapidement tués sur pois : en 8 jours, entre 90 et 100% des adultes sont morts.

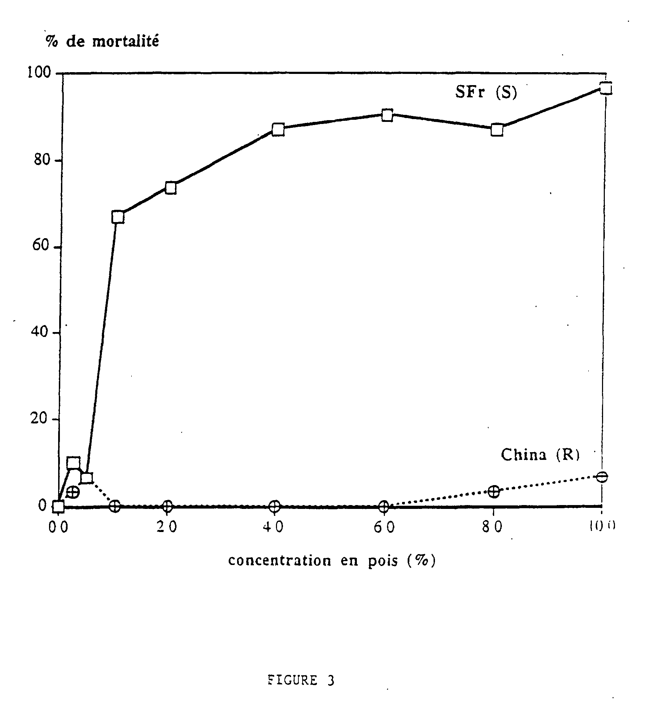

La Figure 3 représente la mortalité à 6 jours de *Sitophilus oryzae* pour des boulettes à différentes concentrations de farine de poids ; la souche résistante (R) et la souche sensible (S) sont comparées. La courbe dose/réponse ainsi établie montre que, pour la souche sensible (S) on observe, dès 10% de farine de poids, 70% de mortalité en 6 jours (et 100% en 14 jours). Dans le même temps, la souche résistante (R) n'est pas affectée.

### Toxicité de farines d'autres légumineuses :

Parmi les graines de légumineuses utilisées en alimentation humaine, 10 ont été testées pour leur action sur les charançons sensibles et résistants.

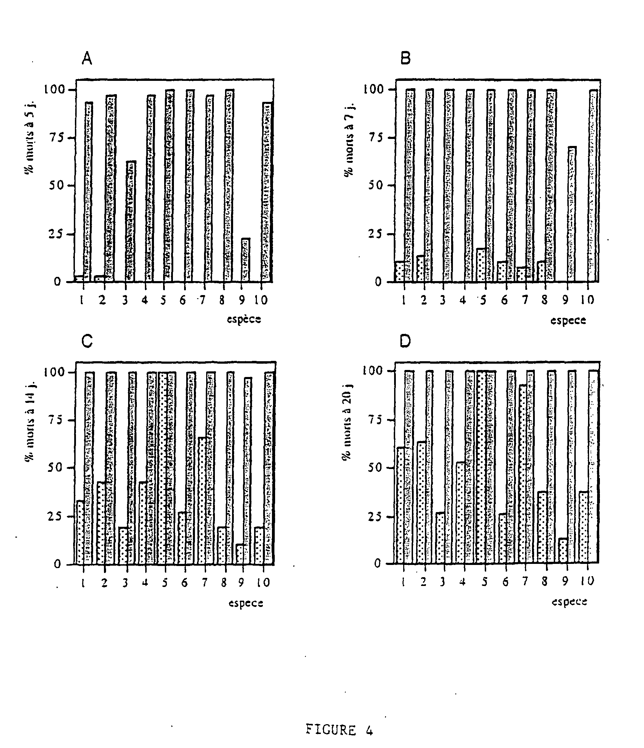

Des boulettes contenant 80% de farine de légumineuse et 20% de farine de blé ont été utilisées. La Figure 4 illustre la mortalité cumulée des charançons *Sitophilus oryzae,* souche résistante R ( 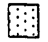 ) ou souche sensible S ( 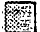 ), mesurée après 5 (4 A), 7 (4 B), 14 (4 C) et 20 jours (4 D) d'alimentation sur niébé (*Vigna unguiculata*) variété blanche (1) et rouge (2), pois de terre (3 : *Vigna subterranea*), lentille (4 : *Lens esculenta*), haricot (5 : *Phaseolus vulgaris*), haricot mungo (6 : *Vigna radiata*), adzuki (7 : *Vigna angularis*)*,* fève (8 : *Vicia faba*), pois chiche (9 : *Cicer arietinum*), et lupin (10 : *Lupinus albus*).

Les résultats montrent qu'à 7 jours toutes les légumineuses sont toxiques pour la souche sensible, même si *Vigna subterranea* et *Cicer arietinum* n'ont pas encore tué tous les insectes qui y vivent ; par contre, la souche résistante ne présente pas ou très peu de mortalité. On peut donc en conclure qu'un même mécanisme à l'origine de la toxicité, est présent chez toutes ces légumineuses ; ce mécanisme apparaît en particulier comme prédominant chez *Vigna subterranea, Vigna radiata* et *Cicer arietinum.*

Toutefois, l'examen des mortalités à 14 et 20 jours sur certaines légumineuses, fait apparaître pour la souche résistante une mortalité plus ou moins grande, qu'il faut donc imputer à d'autres mécanismes ; c'est en particulier le cas sur *Phaseolus vulgaris* et sur *Vigna angularis.*

### EXEMPLE 2 - PURIFICATION ET IDENTIFICATION DE LA SUBSTANCE RESPONSABLE DE LA TOXICITÉ CHEZ LE POIS

### Préparation d'une fraction protéique enrichie en albumines (SRA1).

La fraction enrichie en albumine est préparée à l'échelle pilote selon le protocole développé par CREVIEU et al. [Nahrung, 40(5), pp. 237-244, (1996)].

La farine de pois (10 kg) est mélangée sous agitation avec 140 litres de tampon acétate (pH 4,9), le mélange est centrifugé à 7500 t/min, le surnageant est soumis à une ultrafiltration sur membrane M5, à une température qui ne dépasse pas 25°C. Le rétentat est soumis à une diafiltratibn sur la même membrane, le nouveau rétentat est centrifugé à 6000 t/min pendant 20 min et le surnageant lyophilisé. La poudre obtenue (SRA1) qui représente en moyenne 1% de la masse mise en oeuvre au départ, est utilisée pour les purifications ultérieures.

A chaque étape de la purification, la toxicité des différentes fractions est déterminée selon le protocole décrit à l'exemple 1 ci-dessus.

### Chromatographie d'échange d'anions

10 g de SRA1 sont mis en suspension dans 100 ml d'une solution de méthanol à 60% et agités 1 heure à 4°C. Après centrifugation (30 min, 9000 g, 4°C) le surnageant est récupéré puis le méthanol présent est éliminé à l'évaporateur rotatif. Le volume est alors réajusté à 100 ml par de l'eau et un tampon Tris-HCl 1M (pH 8,8) de manière à obtenir une concentration finale de 50 mM en Tris-HCl. Les protéines solubles sont fractionnées par chromatographie d'échange d'anions sur une colonne (120 x 50 mm) de DEAE SEPHAROSE FAST FLOW. Les protéines adsorbées sont éluées par une concentration de 50% de tampon B (Tris-HCl 50 mM, pH 8,8 ; NaCl 500 mM) dans le tampon A (Tris-HCl 50 mM, pH 8,8). Le débit d'élution est de 20 ml/min et les fractions collectées ont un volume de 80 ml. Les protéines sont détectées par absorption à 280 nm.

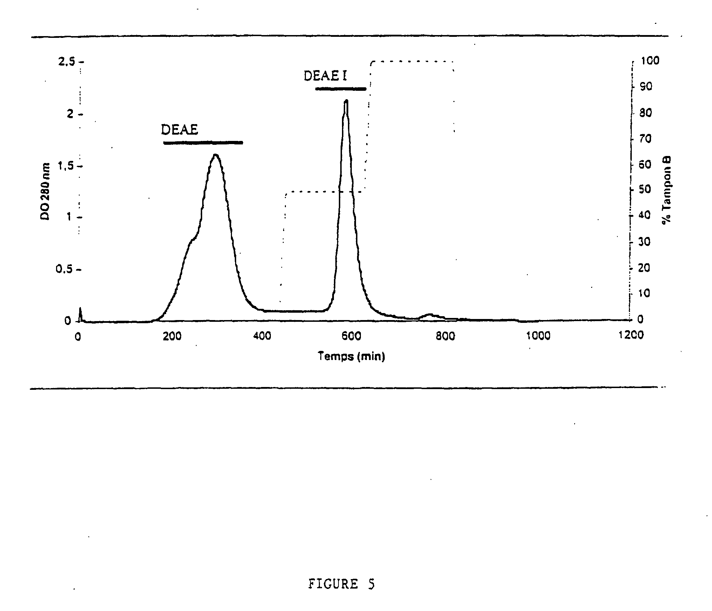

Le chromatogramme est représenté sur la Figure 5. La concentration en tampon B est indiquée par la ligne discontinue. Les fractions de 80 ml correspondant aux pics sont réunies en deux fractions principales, DEAE NA et DEAE 1, indiquées sur le chromatogramme par les lignes horizontales. La fraction non adsorbée (DEAE NA) contient toute la toxicité.

Cette fraction est dialysée 72 heures contre de l'eau puis lyophilisée. On obtient ainsi environ 450 mg de la fraction DEAE NA.

### Chromatographie HPLC phase inverse semi-préparative.

La fraction DEAE NA obtenue après chromatographie échangeuse d'anions est fractionnée par chromatographie HPLC phase inverse (RP-HPLC) sur une colonne HYPERSIL (250 x 10,5 mm) remplie de NUCLÉOSIL 5 µm 300 Å greffé par une chaîne aliphatique en C18. Pour chaque chromatographie, 15 mg de protéines sont déposées sur la colonne Le débit d'élution est de 3 ml/min et les protéines sont détectées par absorption à 220 nm. Les protéines sont éluées par un gradient de tampon B (0,04% d'acide trifluoroacétique dans de l'acétonitrile) dans le mélange A (0,04% d'acide trifluoroacétique dans de l'eau) selon la séquence suivante : t=0 min, 40% de B ; t=5 min, 40% de B ; t=17 min, 48% de B ; t=18 min, 80% de B ; et t=23 min, 80% de B.

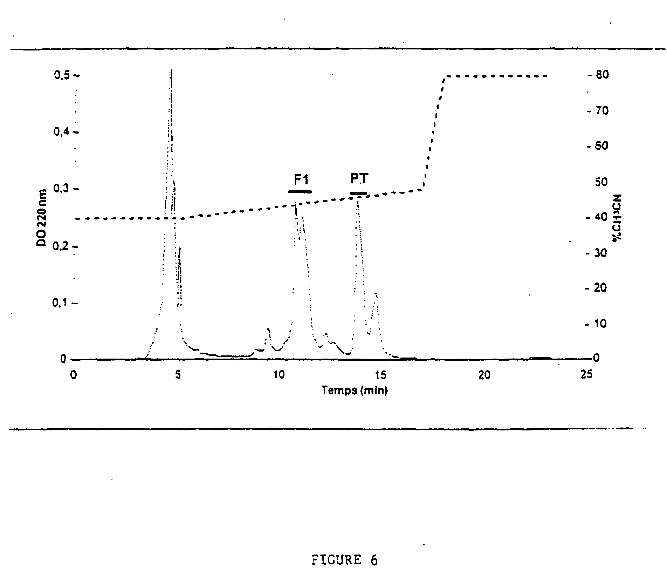

Le chromatogramme est illustré par la Figure 6. Le gradient d'acétonitrile est représenté par la ligne discontinue. La toxicité est localisée uniquement au niveau des pics F1 et PT ; les fractions correspondant à ces pics qui ont été collectées, sont représentées sur le chromatogramme par des par des traits horizontaux,.

Trente chromatographies successives correspondant à une quantité injectée de 450 mg de DEAE NA ont été effectuées. Les fractions ont été réunies puis lyophilisées après évaporation de l'acétonitrile et de l'acide trifluoroacétique au SPEED VAC. 4 mg de la fraction PT et 5 mg de F1 ont ainsi été obtenues. Ces fractions ont ensuite été analysées par chromatographie HPLC phase inverse (RP-HPLC).

### Chromatographie HPLC phase inverse

Le contrôle de la pureté des protéines des fractions F1 et PT est effectué par chromatographie HPLC phase inverse sur une colonne INTERCHROM (250 x 4,6 mm) remplie de NUCLÉOSIL 5 µm 100 Å greffé par une chaîne aliphatique en C18. Le débit d'élution est de 1 ml/min et les protéines sont détectées par absorption à 220 nm.

Les protéines sont éluées en 45 minutes par un gradient linéaire de 0 à 50% de mélange B (0,04% d'acide trifluoroacétique dans de l'acétonitrile) dans le mélange A (0,04% d'acide trifluoroacétique dans de l'eau).

Cette analyse montre que la fraction PT ne contient que la protéine toxique PT. La fraction F1 est plus complexe et contient deux polypeptides majeurs.

### Caractérisation des protéines présentes dans les fractions PT et F1

Les déterminations de masses ont été réalisées par spectrométrie de masse à électrospray (ES-MS). Les masses moyennes calculées à partir de 2 estimations, sont de 3741,1 Da dans le cas de PT, et de 3736 et 3941 Da pour les polypeptides de la fraction FT.

Le nombre de cystéines libres et impliquées dans les ponts disulfures, a été déterminé par alkylation de la protéine par l'iodoacétamide, avant et après réduction, et comparaison des temps de rétention en RP-HPLC et des masses en ES-MS, des protéines alkylées avec la protéine native.

La protéine non-réduite alkylée présente un temps de rétention et une masse identiques à celle de la protéine native. En revanche, la protéine réduite puis alkylée présente un temps de rétention nettement différent de celui observé pour la protéine native (30 min au lieu de 42 min), et une masse de 4089,9 Da.

Il apparaît donc que cette protéine contient 6 cystéines, qui sont toutes impliquées dans 3 ponts disulfures.

### Séquence complète de la protéine PT

La séquence complète de la protéine PT a été établie. La masse calculée à partir des 37 résidus de la protéine est 3741,4 Da, ce qui est identique, à l'erreur de mesure près, à celle déterminée par spectrométrie de masse (3741,1 Da) pour la protéine native. La valeur calculée pour la protéine alkylée par l'iodoacétamide (4090 Da) est également équivalente à celle obtenue expérimentalement (4089,9 Da). Ces résultats démontrent l'absence de modifications post-traductionnelles (glycosylations, phosphorylations...) de la protéine.

La séquence de la protéine PT présente une très forte homologie avec celle de l'albumine de pois PA1b [HIGGINS et al, J. Biol. Chem, 261(24), pp. 11124-11130, (1986)]. Les deux séquences ne diffèrent que par le remplacement du résidu valine en position 29 dans la protéine PT par une isoleucine dans la PA1b. Une forte similitude (62% d'identité, 89% d'homologie, déterminées à l'aide du logiciel MAC MOLLY utilisant la matrice BLOSUM62) est également observée entre la protéine PT et la leginsuline de soja [WATANABE et al., Eur. J. Biochem., 15, pp. 224:1-167-72, (1994)]. En particulier, les 6 résidus cystéine, qui jouent un rôle essentiel dans la structure des protéines, occupent des positions conservées.

La comparaison de ces 3 séquences est illustrée par la figure 7.

Ces résultats permettent de conclure que la protéine responsable de la résistance du pois aux charançons des céréales est similaire à la protéine PA1b décrite par HIGGINS. Cette protéine est synthétisée sous la forme d'une préproprotéine de 130 résidus (PA1) qui subit une maturation post-traductionnelle libérant la protéine PA1b ainsi qu'une protéine de 53 résidus nommée PAla [HIGGINS et al., J. Biol. Chem., 261(24), pp. 11124-11130, (1986)].

Le séquençage des 10 premiers résidus N-terminaux de chacun des polypeptides toxiques de la fraction F1 a également été réalisé. Les séquences obtenues sont identiques à celle de l'extrémité N-terminale de la protéine PT. Comme, d'autre part, les masses de ces polypeptides déterminées par ES-MS sont très proches de celle de la PT, il apparaît que ces polypeptides représentent des isoformes de la PT.

### EXEMPLE 3 - ACTIVITÉ ET STABILITÉ DES PROTÉINES ENTOMOTOXIQUES EXTRAITES DU POIS

### Activité :

L'activité entomotoxique des polypeptides de la fraction PT ou de la fraction F1 a été déterminée comme décrit à l'exemple 1 ci-dessus ; à la concentration de 1% dans la farine de blé (3mmoles/kg), ces polypeptides ont pour le charançon une toxicité équivalente à celle de la farine de pois pure. Une concentration de 60 µmoles/kg est suffisante pour empêcher toute infestation par les charançons.

### Stabilité :

Les polypeptides de la fraction PT ou de la fraction F1, extraits à partir de graines sèches stockées pendant plusieurs années, conservent leur activité entomotoxique. En outre, cette activité n'est pas affectée par un chauffage à 100°C.

### Toxicité pour différents insectes :

La toxicité de la protéine PT pour la teigne de la farine *Ephestia kuehniellea* (Lepidoptera), et pour le puceron *Acyrthosiphon pisum* (Homoptera) a également été testée.

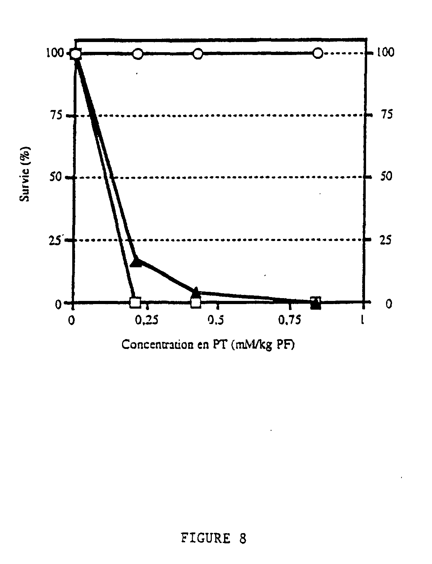

Les essais sur la teigne de la farine ont été effectués sur des larves de premier et deuxième stade d'*Ephestia kuehniella* alimentées sur des boulettes de farine de blé contenant différentes concentrations de la protéine PT (en mmoles par kg de farine de blé). Les résultats sont illustrés par la Figure 8.
(○ = Survie à 0 jour ;
▲ = Survie à 4 jours ;
□ = Survie à 10 jours).

Ces résultats montrent que cette protéine est très toxique, dès la concentration de 0,25mmoles/kg.

Le puceron *Acyrthosiphon pisum* (Homoptera), a été nourri sur milieu artificiel contenant différentes concentrations de la protéine PT.
(□ = 3,3 µM ;
▲ = 17 µM :
◆ = 46 µM ;
○ = 84 µM ;
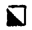 = 100 µM).

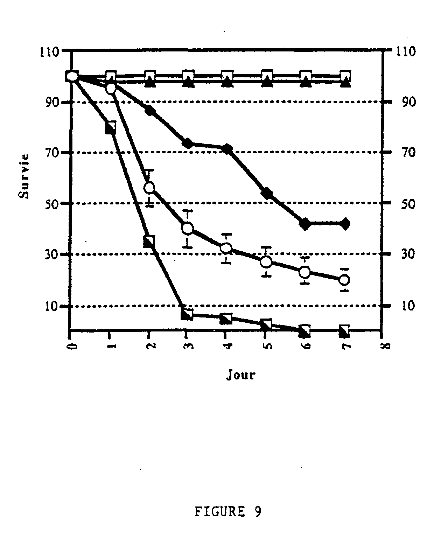

Les résultats, qui sont illustrés par la Figure 9, montrent qu'une importante mortalité apparaît dès la concentration de 46 µmolaire, mortalité qui est totale à 100 µmolaire.

## Revendications

1. Utilisation comme insecticide d'un polypeptide comprenant une séquence répondant à la formule générale (I) suivante :
X₁CX₂CX₃CX₄CX₅CX₆CX₇ (I)
dans laquelle :
- C représente un résidu cystéine,
- X₁ représente la séquence y₁y₂ dans laquelle y₁ et y₂ représentent chacun un acide aminé choisi parmi l'alanine, la sérine, la glycine, et la thréonine, ou bien y₁ représente un acide aminé choisi parmi l'alanine, la sérine, la glycine, et la thréonine, et y₂ représente l'acide glutamique ou l'acide aspartique ;
- X₂ représente la séquence y₃y₄y₅ dans laquelle y₃ représente la glutamine ou l'asparagine, et y₄ et y₅ représentent chacun un acide aminé choisi parmi l'alanine, la sérine, la glycine, la thréonine, la valine, la leucine, l'isoleucine, et la méthionine ;
- X₃ représente la séquence y₆y₇y₈y₉y₁₀y₁₁y₁₂ dans laquelle y₆ représente un acide aminé choisi parmi l'alanine, la sérine, la glycine, et la thréonine, y₇, y₁₁, et y₁₂ représentent chacun la proline, y₈ représente un acide aminé choisi parmi la phénylalanine, le tryptophane, et la tyrosine, y₉ représente l'acide aspartique ou l'acide glutamique, y₁₀ représente un acide aminé choisi parmi la valine, la leucine, l'isoleucine, et la méthionine ;
- X₄ représente la séquence y₁₃y₁₄y₁₅y₁₆, dans laquelle y₁₃, y₁₄, y₁₅, y₁₆, représentent chacun un acide aminé choisi parmi l'alanine, la sérine, la glycine, et la thréonine, ou bien y₁₄ représente un acide aminé choisi parmi l'alanine, la sérine, la glycine, et la thréonine, y₁₃ et y₁₅ représentent chacun un acide aminé basique, et y₁₆ représente l'acide aspartique ou l'acide glutamique ;
- X₅ représente un acide aminé basique ;
- X₆ répond à la séquence y₁₇y₁₈y₁₉y₂₀y₂₁y₂₂y₂₃y₂₄y₂₅, dans laquelle y₁₇, y_{19,} y₂₁, et y₂₃ représentent chacun un acide aminé choisi parmi la valine, la leucine, l'isoleucine, et la méthionine, y₁₈ représente la proline, y₂₀ et y₂₄ représentent chacun un acide aminé choisi parmi l'alanine, la sérine, la glycine, et la thréonine, y₂₂ représente un acide aminé choisi parmi la valine, la leucine, l'isoleucine, la méthionine, la phénylalanine, le tryptophane, et la tyrosine, et y₂₅ représente un acide aminé choisi parmi la phénylalanine, le tryptophane, et la tyrosine ;
- X₇ représente la séquence y₂₆y₂₇y₂₈y₂₉y₃₀ dans laquelle y₂₆ représente un acide aminé basique, ou un acide aminé choisi parmi la valine, la leucine, l'isoleucine, et la méthionine, y₂₇ représente l'asparagine ou la glutamine ou un acide aminé basique, y₂₈ représente la proline, et y₂₉ et y₃₀ représentent chacun un acide aminé choisi parmi l'alanine, la sérine, la glycine, et la thréonine.

2. Utilisation selon la revendication 1, **caractérisée en ce que** ledit polypeptide présente au moins 60% d'identité avec l'albumine PAlb de pois.

3. Utilisation selon la revendication 1, **caractérisé en ce que** ledit polypeptide comprend une séquence de formule (I) définie ci-dessus, dans laquelle :
- y₁ représente l'alanine, y₃ représente l'asparagine, y₄ représente la glycine, y₆ représente la sérine,y₇, y₁₁, et y₁₂ représentent chacun la proline, y₈ représente la phénylalanine, y₉ représente l'acide glutamique, X₅ représente l'arginine, y₁₈ représente la proline, y₂₀ représente la glycine, y₂₁ représente la leucine, y₂₄ représente la glycine, y₂₅ représente la tyrosine, y₂₈ représente la proline, y₃₀ représente la glycine,
et :
- y₂ représente la sérine, y₅ représente la valine, y₁₀ représente la méthionine, y₁₃ représente la glycine, y₁₄ représente la thréonine, y₁₅ représente la sérine, y₁₆ représente l'alanine, y₁₇ représente l'isoleucine, y₁₉ représente la valine, y₂₂ représente la valine, y₂₃ représente l'isoleucine ou la valine, y₂₆ représente l'arginine, y₂₇ représente l'asparagine, y₂₉ représente la sérine,
ou bien :
- y₂ représente l'acide aspartique, y₅ représente l'alanine, y₁₀ représente la valine, y₁₃ représente l'arginine, y₁₄ représente la sérine, y₁₅ représente l'arginine, y₁₆ représente l'acide aspartique, y₁₇ représente la valine, y₁₉ représente l'isoleucine, y₂₂ représente la phénylalanine, y₂₃ représente la valine, y₂₆ représente l'isoleucine, y₂₇ représente l'histidine, y₂₈ représente la proline, et y₂₉ représente la thréonine.

4. Utilisation selon la revendication 1, **caractérisée en ce que** ledit polypeptide est choisi dans le groupe constitué par les albumines PAlb et les leginsulines.

5. Utilisation selon une quelconque des revendications 1 à 4, **caractérisée en ce que** ledit polypeptide est utilisé pour protéger des graines de céréales ou des produits dérivés de celles-ci, contre des insectes ravageurs.

6. Utilisation selon une quelconque des revendications 1 à 4, **caractérisée en ce que** ledit polypeptide est utilisé pour protéger des plantes contre les insectes ravageurs des graines de céréales.

7. Utilisation selon une quelconque des revendications 1 à 6, **caractérisée en ce que** ledit polypeptide est utilisé à une concentration de 10 µmoles/kg à 100 mmoles/kg.

8. Utilisation selon la revendication 7, **caractérisée en ce que** ledit polypeptide est utilisé à une concentration de 50 µmoles/kg à 10 mmoles/kg.

9. Utilisation selon une quelconque des revendications 1 à 8, **caractérisé en ce qu'**elle comprend le traitement du produit à protéger avec une préparation comprenant ledit polypeptide.

10. Utilisation selon une quelconque des revendications 1 à 9, **caractérisée en ce qu'**elle comprend la production d'une plante transgénique, transformée par au moins un gène codant pour ledit polypeptide, et exprimant ce dernier dans au moins un de ses tissus ou organes.

## Patentansprüche

1. Verwendung eines Polypeptids als Insektizid, das eine der nachfolgenden allgemeinen Formel (I) entsprechende Sequenz umfaßt:
X₁CX₂CX₃CX₄CX₅CX₆CX₇ (I)
worin:
C einen Cysteinrest darstellt,
X₁ die Sequenz y₁y₂ darstellt, worin y₁ und y₂ jeweils eine Aminosäure darstellen, ausgewählt aus Alanin, Serin, Glycin und Threonin, oder y₁ eine aus Alanin, Serin, Glycin und Threonin ausgewählte Aminosäure darstellt und y₂ Glutaminsäure oder Aspartamsäure;
X₂ die Sequenz y₃y₄y₅ darstellt, worin y₃ Glutamin oder Asparagin darstellt und y₄ und y₅ jeweils eine aus Alanin, Serin, Glycin, Threonin, Valin, Leucin, Isoleucin und Methionin ausgewählte Aminosäure;
X₃ die Sequenz y₆y₇y₈y₉y₁₀y₁₁y₁₂ darstellt, worin y₆ eine aus Alanin, Serin, Glycin und Threonin ausgewählte Aminosäure darstellt, y₇, y₁₁ und y₁₂ jeweils Prolin, y₈ eine aus Phenylalanin, Tryptophan und Thyrosin ausgewählte Aminosäure, y₉ Aspartamsäure oder Glutaminsäure, y₁₀ eine aus Valin, Leucin, Isoleucin und
Methionin ausgewählte Aminosäure;
X₄ die Sequenz y₁₃y₁₄y₁₅y₁₆ darstellt, worin y₁₃, y₁₄, y₁₅, y₁₆ jeweils eine aus Alanin, Serin, Glycin und Threonin ausgewählte Aminosäure darstellen, oder y₁₄ eine aus Alanin, Serin, Glycin und Threonin ausgewählte Aminosäure, y₁₃ und y₁₅ jeweils eine basische Aminosäure und y₁₆ Aspartamsäure oder Glutaminsäure;
X₅ eine basische Aminosäure darstellt;
X₆ der Sequenz y₁₇y₁₈y₁₉y₂₀y₂₁y₂₂y₂₃y₂₄y₂₅ entspricht, worin y₁₇, y₁₉, y₂₁ und y₂₃ jeweils eine aus Valin, Leucin, Isoleucin und Methionin ausgewählte Aminosäure darstellen, y₁₈ Prolin, y₂₀ und y₂₄ jeweils eine aus Alanin, Serin, Glycin und Threonin ausgewählte Aminosäure, y₂₂ eine aus Valin, Leucin, Isoleucin, Methionin, Phenylalanin, Tryptophan und Tyrosin ausgewählte Aminosäure und y₂₅ eine aus Phenylalanin, Tryptophan und Tyrosin ausgewählte Aminosäure;
X₇ die Sequenz y₂₆y₂₇y₂₈y₂₉y₃₀ darstellt, worin y₂₆ eine basische Aminosäure darstellt oder eine aus Valin, Leucin, Isoleucin und Methionin ausgewählte Aminosäure, y₂₇ Asparagin oder Glutamin oder eine basische Aminosäure, y₂₈ Prolin und y₂₉ und y₃₀ jeweils eine aus Alanin, Serin, Glycin und Threonin ausgewählte Aminosäure.

2. Verwendung gemäß Anspruch 1, **dadurch gekennzeichnet, daß** das genannte Polypeptid eine wenigstens 60%ige Identität mit dem Erbsenalbumin PA1b zeigt.

3. Verwendung gemäß Anspruch 1, **dadurch gekennzeichnet, daß** das genannte Polypeptid eine oben definierte Sequenz der Formel (I) umfaßt, worin:
y₁ Alanin darstellt, y₃ Asparagin, y₄ Glycin, y₆ Serin, y₇, y₁₁ und y₁₂ jeweils Prolin, y₈ Phenylalanin, y₉ Glutaminsäure, x₅ Arginin, y₁₈ Prolin, y₂₀ Glycin, y₂₁ Leucin, y₂₄ Glycin, y₂₅ Tyrosin, y₂₈ Prolin, y₃₀ Glycin,
und:
y₂ Serin darstellt, y₅ Valin, y₁₀ Methionin, y₁₃ Glycin, y₁₄ Threonin, y₁₅ Serin, y₁₆ Alanin, y₁₇ Isoleucin, y₁₉ Valin, y₂₂ Valin, y₂₃ Isoleucin oder Valin, y₂₆ Arginin, y₂₇ Asparagin, y₂₉ Serin,
oder:
y₂ Aspartamsäure darstellt, y₅ Alanin, y₁₀ Valin, y₁₃ Arginin, y₁₄ Serin, y₁₅ Arginin, y₁₆ Aspartamsäure, y₁₇ Valin, y₁₉ Isoleucin, y₂₂ Phenylalanin, y₂₃ Valin, y₂₆ Isoleucin, y₂₇ Histidin, y₂₈ Prolin und y₂₉ Threonin.

4. Verwendung gemäß Anspruch 1, **dadurch gekennzeichnet, daß** das genannte Polypeptid aus der durch die Albumine PA1b und den Leginsulinen gebildete Gruppe ausgewählt ist.

5. Verwendung gemäß einem der Ansprüche 1 bis 4, **dadurch gekennzeichnet, daß** das genannte Polypeptid zum Schutz von Getreidekömern oder von diesen abgeleiteten Produkten gegen Schadinsekten verwendet wird.

6. Verwendung gemäß einem der Ansprüche 1 bis 4, **dadurch gekennzeichnet, daß** das genannte Polypeptid zum Schutz von Pflanzen gegen Getreidekörnerschadinsekten verwendet wird.

7. Verwendung gemäß einem der Ansprüche 1 bis 6, **dadurch gekennzeichnet, daß** das genannte Polypeptid in einer Konzentration von 10 µmol/kg bis 100 mmol/kg verwendet wird.

8. Verwendung gemäß Anspruch 7, **dadurch gekennzeichnet, daß** das genannte Polypeptid in einer Konzentration von 50 µmol/kg bis 10 mmol/kg verwendet wird.

9. Verwendung gemäß einem der Ansprüche 1 bis 8, **dadurch gekennzeichnet, daß** sie die Behandlung des zu schützenden Produkts mit einer das genannte Polypeptid umfassenden Zubereitung umfaßt.

10. Verwendung gemäß einem der Ansprüche 1 bis 9, **dadurch gekennzeichnet, daß** sie die Herstellung einer transgenen Pflanze umfaßt, welche in wenigstens einem, für das genannte Polypeptid kodierenden Gen transformiert wurde, und dem Exprimieren von letzterem in wenigstens einer der Gewebe oder Organe derselben.

## Claims

1. Use, as an insecticide, of a polypeptide comprising a sequence which satisfies the following general formula (I):
X₁CX₂CX₃CX₄CX₅CX₆CX₇ (I)
in which:
- C represents a cysteine residue,
- X₁ represents the sequence y₁y₂ in which y₁ and y₂ each represent an amino acid chosen from alanine, serine, glycine and threonine, or y₁ represents an amino acid chosen from alanine, serine, glycine and threonine, and y₂ represents glutamic acid or aspartic acid;
- X₂ represents the sequence y₃y₄y₅ in which y₃ represents glutamine or asparagine, and y₄ and y₅ each represent an amino acid chosen from alanine, serine, glycine, threonine, valine, leucine, isoleucine and methionine;
- X₃ represents the sequence y₆y₇y₈y₉y₁₀y₁₁y₁₂ in which y₆ represents an amino acid chosen from alanine, serine, glycine and threonine, y₇, y₁₁ and y₁₂ each represent proline, y₈ represents an amino acid chosen from phenylalanine, tryptophan and tyrosine, y₉ represents aspartic acid or glutamic acid, and y₁₀ represents an amino acid chosen from valine, leucine, isoleucine and methionine;
- X₄ represents the sequence y₁₃y₁₄y₁₅y₁₆, in which y₁₃, y₁₄, y₁₅ and y₁₆ each represent an amino acid chosen from alanine, serine, glycine and threonine, or y₁₄ represents an amino acid chosen from alanine, serine, glycine and threonine, y₁₃ and y₁₅ each represent a basic amino acid, and y₁₆ represents aspartic acid or glutamic acid;
- X₅ represents a basic amino acid;
- X₆ satisfies the sequence y₁₇y₁₈y₁₉y₂₀y₂₁y₂₂y₂₃y₂₄y₂₅, in which y₁₇, y₁₉, y₂₁ and y₂₃ each represent an amino acid chosen from valine, leucine, isoleucine and methionine, y₁₈ represents proline, y₂₀ and y₂₄ each represent an amino acid chosen from alanine, serine, glycine and threonine, y₂₂ represents an amino acid chosen from valine, leucine, isoleucine, methionine, phenylalanine, tryptophan and tyrosine, and y₂₅ represents an amino acid chosen from phenylalanine, tryptophan and tyrosine;
- X₇ represents the sequence y₂₆y₂₇y₂₈y₂₉y₃₀ in which y₂₆ represents a basic amino acid or an amino acid chosen from valine, leucine, isoleucine and methionine, y₂₇ represents asparagine or glutamine or a basic amino acid, y₂₈ represents proline, and y₂₉ and y₃₀ each represent an amino acid chosen from alanine, serine, glycine and threonine.

2. Use according to Claim 1, **characterized in that** said polypeptide has at least 60% identity with pea PA1b albumin.

3. Use according to Claim 1, **characterized in that** said polypeptide comprises a sequence of formula (I) defined above, in which:
- y₁ represents alanine, y₃ represents asparagine, y₄ represents glycine, y₆ represents serine, y₇, y₁₁ and y₁₂ each represent proline, y₈ represents phenylalanine, y₉ represents glutamic acid, X₅ represents arginine, y₁₈ represents proline, y₂₀ represents glycine, y₂₁ represents leucine, y₂₄ represents glycine, y₂₅ represents tyrosine, y₂₈ represents proline, and y₃₀ represents glycine,
and:
- y₂ represents serine, y₅ represents valine, y₁₀ represents methionine, y₁₃ represents glycine, y₁₄ represents threonine, y₁₅ represents serine, y₁₆ represents alanine, y₁₇ represents isoleucine, y₁₉ represents valine, y₂₂ represents valine, y₂₃ represents isoleucine or valine, y₂₆ represents arginine, y₂₇ represents asparagine, and y₂₉ represents serine,
or else:
- y₂ represents aspartic acid, y₅ represents alanine, y₁₀ represents valine, y₁₃ represents arginine, y₁₄ represents serine, y₁₅ represents arginine, y₁₆ represents aspartic acid, y₁₇ represents valine, y₁₉ represents isoleucine, y₂₂ represents phenylalanine, y₂₃ represents valine, y₂₆ represents isoleucine, y₂₇ represents histidine, y₂₈ represents proline, and y₂₉ represents threonine.

4. Use according to Claim 1, **characterized in that** said polypeptide is chosen from the group consisting of PA1b albumins and leginsulins.

5. Use according to any one of Claims 1 to 4, **characterized in that** said polypeptide is used for protecting cereal seeds, or products derived from them, against insect pests.

6. Use according to any one of Claims 1 to 4, **characterized in that** said polypeptide is used for protecting plants against insects which are pests for cereal seeds.

7. Use according to any one of Claims 1 to 6, **characterized in that** said polypeptide is used at a concentration of 10 µmol/kg to 100 mmol/kg.

8. Use according to Claim 7, **characterized in that** said polypeptide is used at a concentration of 50 µmol/kg to 10 mmol/kg.

9. Use according to any one of Claims 1 to 8, **characterized in that** it comprises the treatment of the product to be protected with a preparation comprising said polypeptide.

10. Use according to any one of Claims 1 to 9, **characterized in that** it comprises the production of a transgenic plant which is transformed with at least one gene encoding said polypeptide, and which expresses the latter in at least one of its tissues or organs.
